# EUROPEAN PATENT SPECIFICATION

(11) **EP 1 912 928 B1**
(45) Date of publication and mention of the grant of the patent: **26.03.2014**
(21) Application number: 06800421.7
(22) Date of filing: 27.07.2006
(51) Int. Cl.: C07C 51/47, C07C 51/44, C07C 63/26, C07C 63/15

(54) **PROCESS FOR REMOVAL OF BENZOIC ACID FROM AN OXIDIZER PURGE STREAM**
VERFAHREN ZUR ENTFERNUNG VON BENZOESÄURE AUS EINEM OXIDATIONSMITTELSÄUBERUNGSSTROM
PROCEDE D'ELIMINATION D'ACIDE BENZOIQUE PROVENANT D'UN FLUX DE PURGE D'UN OXYDANT

(30) Priority: 11.08.2005 US 201512; 11.08.2005 US 201799
(43) Date of publication of application: 23.04.2008
(73) Proprietor: GRUPO PETROTEMEX, S.A. DE C.V., San Pedro Garza Garcia, Nuevo Leon 66265 (MX)
(72) Inventor: LIN, Robert, Kingsport, TN 37664 (US); GIBSON, Philip, Edward, Kingsport, TN 37660 (US); PARKER, Kenny, Randolph, Afton, TN 37616 (US)
(74) Representative: Tostmann, Holger Carl
(86) International application number: PCT/US2006/029268
(87) International publication number: WO 2007/021488

(56) References cited:
- WO-A-97/30963
- GB-A- 2 067 563

## Description

### FIELD OF INVENTION

This invention relates to the removal of impurities, specifically benzoic acid, from a mother liquor produced in the synthesis of carboxylic acid, typically terephthalic acid. This invention also relates to the removal of impurities, specifically benzoic acid, from a benzoic acid bearing stream produced in the synthesis of carboxylic acid.

### BACKGROUND OF THE INVENTION

Terephthalic acid is commercially produced by oxidation of paraxylene in the presence of a catalyst, such as, for example, Co, Mn, Br and a solvent. Terephthalic acid used in the production of polyester fibers, films, and resins must be further treated to remove impurities formed as a result of the oxidation of paraxylene.

Terephthalic acid (TPA) is an intermediate in the production of polyesters for plastics and fiber applications. Commercial processes for the manufacture of TPA are often based on the heavy-metal catalyzed oxidation of p-xylene, generally with a bromide promoter in an acetic acid solvent. Due to the limited solubility of TPA in acetic acid under practical oxidation conditions, a slurry of TPA crystals is usually formed in the oxidation reactor. Typically, the TPA oxidizer slurry is withdrawn from the reactor, and TPA solids are separated from the oxidizer mother liquor using conventional solid-liquid separation techniques. The oxidizer mother liquor, which contains most of the catalyst and promoter used in the process, is recycled to the oxidation reactor. Aside from the catalyst and promoter, the oxidizer mother liquor stream also contains dissolved TPA and many by-products and impurities. These by-products and impurities arise partially from minor impurities present in the p-xylene feed stream. Other impurities arise due to the incomplete oxidation of p-xylene resulting in partially oxidized products. Still other by-products result from competing side reactions formed as a result of the oxidation of p-xylene to terephthalic acid.

The TPA solids undergo a solid-liquid separation wherein fresh solvent is utilitized to displace a major portion of the liquid component of the oxidizer mother liquor. After drying, the TPA solids are contaminated with impurities that were present in the oxidizer mother liquor since these impurities may be incorporated into the TPA solids. Impurities are also present due to occlusions in the TPA crystal structure and due to incomplete removal of the oxidizer mother liquor by the fresh solvent wash.

Many of the impurities in the oxidizer mother liquor stream that are recycled are relatively inert to further oxidation. Such impurities include, for example, isophthalic acid, phthalic acid and trimellitic acid. Impurities, which may undergo further oxidation are also present, such as, for example, 4-carboxybenzaldehyde, p-toluic acid and p-tolualdehyde. Oxidation inert impurities tend to accumulate in the oxidizer mother liquor upon recycle. The concentration of these inert impurities will increase in the oxidizer mother liquor until an equilibria is reached whereby the rate of removal of each impurity via the TPA product balances with the rate of formation and the rate of addition to the oxidation process. The normal level of impurities in commercial crude TPA makes it unsuitable for direct use in most polymer applications.

Conventionally, crude TPA has been purified either by conversion of a dimethyl ester or by dissolution in water with subsequent hydrogenation over standard hydrogenation catalysts. More recently, secondary oxidative treatments have been used to produce polymer-grade TPA. It is desirable to minimize the concentration of impurities in the mother liquor and thereby facilitate subsequent purification of TPA. In some cases, it is not possible to produce a purified, polymer-grade TPA unless some means for removing impurities from the oxidizer mother liquor stream are utilized.

WO 9730963 (A1) discloses a process for the production of an aromatic carboxylic acid comprising oxidising a precursor of the aromatic carboxylic acid in an aqueous liquid phase medium comprising a lower aliphatic carboxylic acid and in the presence of a heavy metal catalyst system, withdrawing from the reaction mixture a slurry of the aromatic carboxylic acid in mother liquor comprising mainly the aliphatic acid, subjecting the slurry to a solids-liquid separation to recover crystals of aromatic carboxylic acid, recycling a first fraction of the resulting mother liquor to the oxidation reaction, concentrating a second fraction of the separated mother liquor to remove aliphatic acid, and disposing of or processing the concentrated residue, characterised in that the second fraction of mother liquor is subjected to solids-liquid separation prior to concentration thereof.

GB 2067563 (A) discloses a process for the synthesis of terephthalic acid comprising oxidizing p-xylene with oxygen in an acetic acid solution in the presence of a catalyst system, separating solid terephthalic acid from the mother reaction liquor, treating the mother liquor to reduce the water content thereof and concentrating at least a portion of the resulting liquor to remove from 70 to 90% of the 25 acetic acid contained therein, in which said concentrated mother liquor is cooled below WC to cause precipitation of a solid phase, the solid phase is separated from the remaining liquid phase containing organic impurities and at least a portion of the solid phase recycled to the synthesis zone; the remaining liquid phase is extracted with water or aqueous-acetic acid solution in the presence of a coadjuvant selected from p-xylene, isobutyl acetate and secondary 30 butyl acetate to form a heavy phase which separates and at least a portion of the heavy phase is recycled to the synthesis zone.

One technique for impurity removal from a recycle stream commonly used in the chemical processing industry is to draw out or "purge" some portion of the recycle stream. Typically, the purge stream is simply disposed of or, if economically justified, subjected to various treatments to remove undesired impurities while recovering valuable components. One example of this process is U.S. # 4,939,297. The amount of purge required for control of impurities is process-dependent; however, a purge amount equal to 10-40 wt% of the total oxidizer mother liquor stream is usually sufficient to produce TPA adequate as feedstock for commercial polymer manufacture. In the production of TPA, the percentage of the oxidizer mother liquor stream purge necessary to maintain acceptable impurity concentrations, coupled with the economic value of the metal catalyst and solvent components in the oxidizer purge stream, make simple disposal of the oxidizer purge stream economically unattractive. Thus, there is a need for a process that recovers essentially all of the valuable metal catalysts and acetic acid contained in the oxidizer purge stream while removing a major portion of the impurities present in the oxidizer purge stream. The metal catalyst can be recovered in an active form suitable for reuse by direct recycling to the p-xylene oxidation step.

One benefit of this invention is the energy and capital cost savings compared with the extraction based purge process previously described.

Another benefit of this invention is its efficacy compared with extraction purge processes regarding the usefulness of the solvent stream(s) recycled to the TPA process. The primary motivation in a liquid extraction process is based upon the assumption that introducing any aromatic impurities into a p-xylene oxidation process for producing terephthalic acid has a detrimental effect on the terephthalic acid powder quality (e.g. yellow color). Hence, it was assumed that a broad spectrum removal of aromatic impurities, such as provided by liquid extraction, was necessary to achieve appropriate terephthalic acid powder quality.

In one embodiment of this invention, however, employs a relatively simple process that separates benzoic acid from an aqueous solvent. The efficiency of the process toward benzoic acid is high since benzoic acid is more volatile (a higher vapor pressure) than most identified aromatic impurities in the production of a carboxylic acid, typically terephthalic acid. These aromatic impurities include, trimellitic acid, isophthalic acid, stilbenes, and anthraquinones. Therefore, it is rather surprising that removal of a benzoic acid in favor of the other known impurities, that are inherently colored, would be sufficient to produce a carboxylic acid, typically terephthalic acid of good quality.

### SUMMARY OF THE INVENTION

In a first embodiment of this invention, a process to produce a benzoic acid stream is provided. The process comprises:
(a) subjecting an oxidizer purge stream to evaporation in a main evaporator zone to produce a vapor stream and a super concentrated purge slurry;
(b) filtering the super concentrated purge slurry in a solid-liquid separation zone to form a filter cake and a mother liquor wherein said solid-liquid separation zone comprises at least one device selected from the group consisting of pressure belt filters, filter presses, centrifuges, pressure leaf filters, and cross-flow filters;
(c) washing the filter cake with a wash feed in said solid-liquid separation zone to form a washed cake and a wash filtrate;
(d) subjecting the mother liquor to evaporation in a evaporator zone to form a solvent rich vapor; and
(e) subjecting the solvent rich vapor to distillation in a separation zone to form a solvent rich stream and a benzoic acid rich stream.

In another embodiment of this invention, a process to produce a benzoic acid stream is provided.

The process comprises:
(a1) subjecting an oxidizer purge stream to evaporation in a first evaporator zone to produce a vapor stream and a concentrated purge slurry;
(a2) subjecting the concentrated purge slurry to evaporation in a second evaporator zone to form a solvent rich stream and a super concentrated purge slurry;
(b) filtering a super concentrated purge slurry in a solid-liquid separation zone to form a filter cake and a mother liquor wherein said solid-liquid separation zone comprises at least one device selected from the group consisting of pressure belt filters, filter presses, centrifuges, pressure leaf filters, and cross-flow filters;
(c) washing the filter cake with a wash feed in the solid-liquid separation zone to form a washed cake and a wash filtrate; wherein the solid- liquid separation zone comprises at least one pressure filtration device;
(d) subjecting the mother liquor to evaporation in a evaporator zone to from a solvent rich vapor; and
(e) subjecting the solvent rich vapor to distillation in a separation zone to form a solvent rich stream and a benzoic acid rich stream; wherein the benzoic acid rich stream comprises at least 60% by weight benzoic acid.

### BRIEF DESCRIPTION OF THE DRAWINGS

Figure 1 illustrates different embodiments of the invention wherein a process to recover benzoic acid from an oxidizer purge stream **101** is shown.
Figure 2 illustrates an embodiment of the process occurring in the solid-liquid separation zone **151** wherein the solid-liquid separation zone comprises a filtration zone **153**, a washing zone **155**, and optionally a dewatering zone **157**.
Figure 3 illustrates an embodiment of the invention where a rotary pressure drum filter is utilized in the solid-liquid separation zone.
Figure 4 illustrates an embodiment of the invention wherein a process to recover benzoic acid from an oxidizer purge stream **101** is shown, and the process utilizes a main evaporator zone **125**.
Figure 5 illustrates an embodiment not according to the invention wherein a process to recovery benzoic acid from a benzoic acid bearing stream **347** is provided.

### DESCRIPTION OIF THE INVENTION:

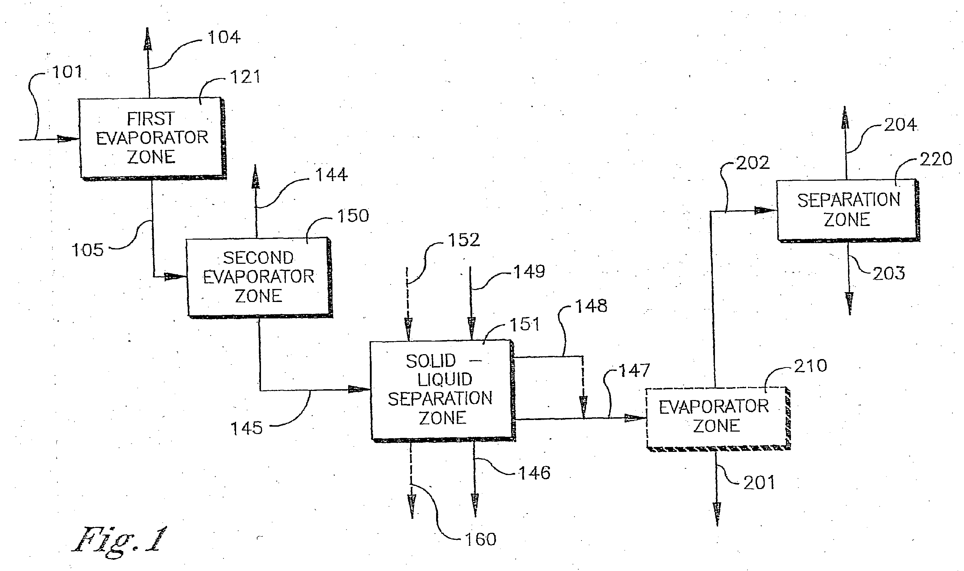

In one embodiment of this invention, a process to separate benzoic acid from an oxidizer purge stream **101** is provided as shown in Figure 1.

Step (a) comprises subjecting an oxidizer purge stream **101** to evaporation in a first evaporator zone **121** to produce a vapor stream **104** and a concentrated purge slurry **105**.

In an embodiment of the invention, the oxidizer purge stream **101** is withdrawn from a carboxylic acid oxidative synthesis process. The oxidizer purge stream **101** serves as the feed stream to the present process. In an embodiment of the invention, the oxidizer purge stream **101** comprises at least one carboxylic acid, at least one solvent, at least one metal catalyst and impurities. The impurities comprise at least one impurity selected from the group consisting of organic bromides, corrosion metals, p-xylene oxidation by-products, and impurities derived as a result of impurities in the p-xylene. The organic bromides may be used as promoters in the oxidation reaction. Examples of corrosion metals are iron and chromium compounds, which inhibit, reduce or entirely destroy the activity of the metal catalyst. Aside from the catalyst and promoter, the oxidizer purge stream **101** also contains by-products and impurities. These by-products and impurities arise partially from minor impurities present in the p-xylene feed stream. Other impurities arise due to the incomplete oxidation of p-xylene resulting in partially oxidized products. Still other by-products result from competing side reactions in the oxidation of p-xylene to terephthalic acid.

The carboxylic acids include any aromatic carboxylic acids produced via controlled oxidation of an organic substrate. Such aromatic carboxylic acids include compounds with at least one carboxylic acid group attached to a carbon atom that is part of an aromatic ring, preferably having at least 6 carbon atoms, even more preferably having only carbon atoms. Suitable examples of such aromatic rings include, benzene, biphenyl, terphenyl, naphthalene, and other carbon-based fused aromatic rings. Examples of suitable carboxylic acids include, terephthalic acid, benzoic acid, p-toluic acid, isophthalic acid, trimellitic acid, naphthalene dicarboxylic acid, 2,5-diphenyl-terephthalic acid, and mixtures thereof.

Suitable solvents include, aliphatic mono-carboxylic acids, preferably containing 2 to 6 carbon atoms, or benzoic acid and mixtures thereof and mixtures of these compounds with water. Preferably, the solvent is acetic acid mixed with water in a ratio of about 5:1 to about 25:1, preferably between about 8:1 and about 20:1. Throughout the specification, acetic acid will be referred to as the solvent. However, it should be appreciated that other suitable solvents, such as those disclosed previously, may also be utilized.

In the first step of the present process, the oxidizer purge stream **101** is concentrated by conventional means in a first evaporator zone **121** comprising at least one evaporator to produce a vapor stream **104** and a concentrated purge slurry **105**. In an embodiment to the invention, the evaporator is operated at atmospheric or slightly super atmospheric conditions, generally from 1x10⁵ Pa (1 atmosphere) to 1x10⁶ Pa (10 atmospheres). The vapor stream **104** comprises a majority of the water and solvent, and the concentrated purge slurry **105** comprises the remainder of the water and solvent not removed from the oxidizer purge stream **101**. As used herein "majority" means greater than 50% by weight. In an embodiment of the invention, the evaporation removes 50 wt%(weight percent) to 80 wt% of the solvent and water, typically acetic acid and water, which are present in the oxidizer purge stream 101.

Step (a1) comprising subjecting the concentrated purge slurry **105** to evaporation in a second evaporator zone 150 to produce a solvent rich stream **144** and a super concentrated purge slurry **145**.

In an embodiment of the invention, the second evaporator zone **150** comprises at least one evaporator operated at vacuum conditions. In an embodiment of the invention, the evaporation can be conducted at a temperature from 20°C to 70°C; another range is from 30°C to 50°C. In an embodiment of the invention, the combination of evaporators **121** and **150** is operated so as to concentrate the oxidizer purge stream as represented by stream **101** to a condition wherein 75 wt% to 99 wt% of the solvent and water, typically acetic acid and water, are removed from the oxidizer purge stream **101** to produce the super concentrated purge slurry **145**. In another embodiment of the invention another range for operation of the combination of evaporators **121** and **150** is operated so as to concentrate the oxidizer purge stream as represented by stream **101** to a condition wherein 85 wt% to 99 wt% of the solvent and water, typically acetic acid and water, is removed from the oxidizer purge stream **101** to produce the super concentrated purge slurry **145**.

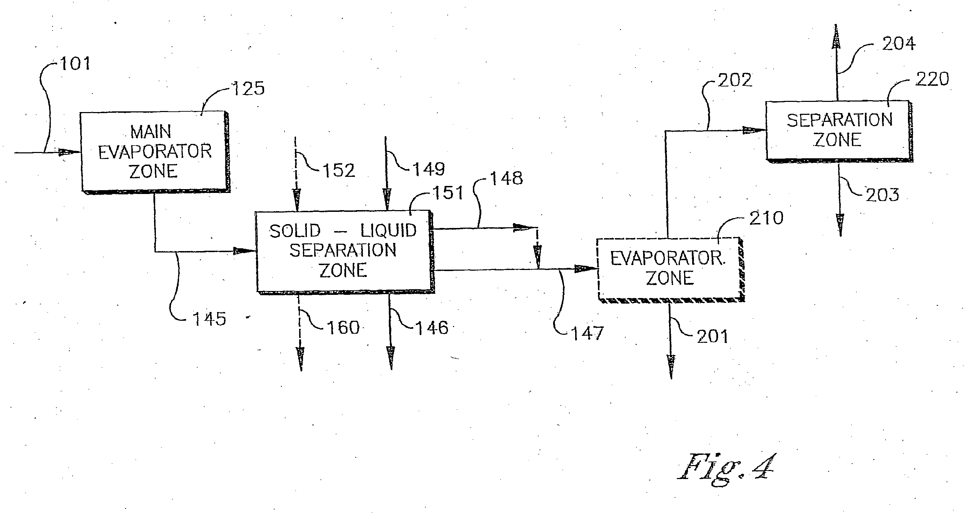

In another embodiment of the invention, the first evaporation zone **121** and the second evaporator zone can be combined in a main evaporation zone **125** as shown in Figure 4. The main evaporation zone **125** comprises at least one evaporator. The evaporator or evaporators in the main evaporation zone **125** are operated at a temperature and pressure sufficient to remove at least 75% by weight of the solvent and water combined from the oxidizer purge stream **101**. In another embodiment of the invention, the evaporator or evaporators in the main evaporation zone **125** are operated at a temperature and pressure sufficient to remove at least 85% by weight of the solvent and water combined from the oxidizer purge stream. In another embodiment of the invention, the evaporator or evaporators in the main evaporation zone **125** are operated at a temperature and pressure sufficient to remove at least 90% by weight of the solvent and water combined from the oxidizer purge stream **101**. In another embodiment of the invention, the evaporator or evaporators in the main evaporation zone **125** are operated at a temperature and pressure sufficient to remove at least 95% by weight of the solvent and water combined from the oxidizer purge stream.

Ranges stated in this disclosure and the claims that follow should be understood to disclose the entire range specifically and not just the end point(s). For example, disclosure of the range 0 to 10 should be taken to specifically disclose 2, 2.5, 3.17 and all other number subsumed and not just 0 and 10.

In an embodiment of the invention, the condition of the super concentrated purge slurry **145** can be as a solid-liquid mixture with only enough solvent to provide pumpability.

Step (b) comprises filtering the super concentrated purge slurry **145** in a solid-liquid separation zone **151** to form a filter cake **154** and a mother liquor **147** wherein said solid-liquid separation zone comprises at least one device selected from the group consisting of pressure belt filters, filter presses, centrifuges, pressure leaf filters, and cross-flow filters.

Step (c) comprises washing the filter cake **154** with a wash feed **149** in the solid-liquid separation zone **151** to form a washed cake **146** and a wash filtrate **148**; wherein the solid-liquid separation zone **151** comprises at least one pressure filtration device.

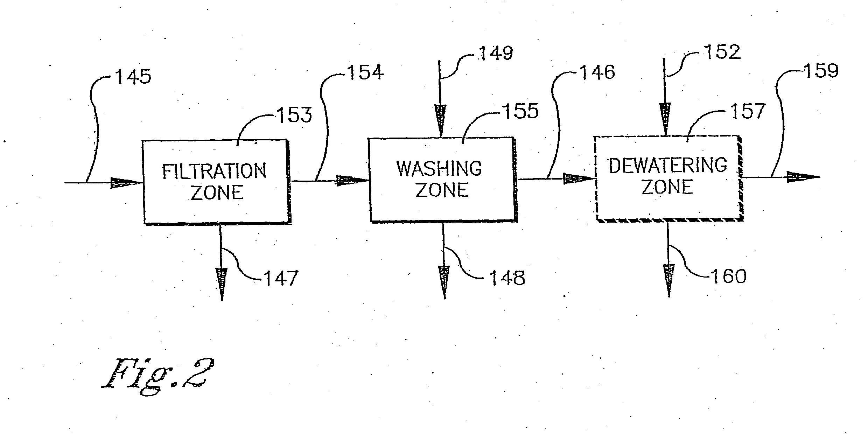

In an embodiment of the invention, the super concentrated purge slurry **145** is introduced in the solid-liquid separation zone **151** where the solid-liquid separation zone comprises a filtration zone **153**, a washing zone **155**, and optionally a drying zone **157** as shown in Figure 2. The filtration zone **153** comprises a filter cell, or a series of filter cells, physically situated to permit a filter cake **154** to develop a distribution across the area of the filter cell to hinder or prevent the channeling of wash feed **149** through the filter cake **154**.

Suitably, a filter cake **154** of at least 0.64 cm (0.25 inch) in depth to 20 cm (8 inches) in depth, preferably at least 1.3 cm (0.5 inch) in depth, more preferably at least 3 cm (1 inch) in depth, and even more preferably about 5 to about 10 cm (2 to 4 inches) in depth is distributed over the area of the filter cell. The washed cake, **146**, can be recovered or further treated, recycled and/or sent to waste treatment facilities.

Upon obtaining a suitable or preferred height of filter cake **154** the filter cake **154** leaves the filtration zone **153** which comprises a filter or series of filters and enters a washing zone 155 where the filter cake 154 is contacted with a wash feed **149**. In one embodiment of the invention, there is sufficient pressure across the filter cake **154** to allow a reservoir or buildup of the wash feed **149** over the filter cake 154 to a suitable depth, preferably to a minimum depth of 0.64 cm (0.25 inch). A pressure gradient of at least 3.4 kPa (0.5 psi), preferably from 3x10⁴ Pa (5 psi) to 45x10⁴ Pa (65 psi), across the filter cake **154** and the reservoir of wash feed **149** can be applied to displace any solute in the filter cake **154** with wash feed **149**.

A filter cake **154** depth of at least 1.3 cm (0.5 inch) is suitable to obtain a filter cake **154** of sufficient compactness to furnish a wash vehicle, i.e. the filter cake **154**, from which a wash filtrate **148** containing a solute from the filter cake **154** can be removed efficiently by displacement washing. If the filter cake depth **154** is less than 0.64 cm (0.25 inch), channeling of wash feed **149** in the filter cake **154** can occur resulting in non-uniform washing of the filter cake **154**.

Because of the loss of efficiency in displacement washing of the filter cake **154**, a minimum filter cake **154** depth of at least 0.64 cm (0.25 inch) of purified terephthalic acid is preferred.

A minimum liquid height above the filter cake **154** surface is required to ensure that displacement washing occurs. This height must be sufficient to ensure that the filter cake **154** surface is completely covered with wash feed **149**. If the filter cake **154** surface is not covered with wash feed **149**, bypassing of the wash feed **149** can occur without adequate displacement of the solute in the filter cake **154**. Because of irregularities in the filter cake **154** surface, a minimum liquid height of 0.64 cm (0.25 inch) is preferred above the filter cake 154 surface.

It has been found that displacement of the solute from the filter cake **154** using the wash feed **149** at high pressure permits an efficient separation of catalyst metals from the filter cake **154**. Another benefit of high pressure is the reduction of wash feed 149 required to recover cobalt as shown in the examples.

Utilization of added stages in the solid-liquid separation zone **151** can decrease the amount of wash feed **149** required to reduce the total amount of metal catalyst retained in the filter cake **154**. It is convenient therefore that a suitable number of stages of positive displacement washing be used to minimize total wash feed **149** used in displacement washing to reduce need for downstream waste treatment facilities.

It is understood that multiple stages of the displacement washing procedure can replace a single stage displacement washing procedure wherein the quantity of wash feed **149** is sufficient to obtain at least 80 wt% recovery of the metal catalyst from the super concentrated slurry **145** to the mother liquor **147** and the wash filtrate **148**. Additionally, a procedure utilizing multiple stages of counter-current washing can be useful if reduction of the amount of wash feed **149** is determined to be advantageous.

In the process of the instant invention, a super concentrated purge slurry **145** is introduced into one or more of a series of filter cells physically situated to permit a filter cake **154** of requisite thickness to develop.
Upon obtaining a minimum height of filter cake **154**, about 0.64 cm to about 10 cm (0.25 to 4 inches), the filter cake **154** leaves the filter or series of filters and enters a washing zone **155** where the filter cake **154** is washed with a wash feed **149**. Pressure can then be applied to the wash feed **149** to displace the solute (i.e. the liquid and any dissolved compounds such as metal catalyst in the filter cake) of the filter cake **154**. Upon displacement of the solute with the wash feed, the filter cake **154** can be discharged from the filtration zone **155** by any suitable means, and the cycle repeated. In an embodiment of the invention, the ratio of wash feed **149** to filter cake **154** discharge is within the range of from about 1:20 to about 20:1 to reduce the level of metal catalyst in the filter cake by greater than 95 wt%.

Equipment for performing the requisite washing cycle can comprise a series of filter cells maintained in a suitable position to permit a wash feed **149** reservoir to develop over the filter cells. In one embodiment of the invention, suitable equipment can comprise a rotary drum pressure filter with multiple filter cells, fitted with a means for discharging washed cake **146** from the filter cells. The filter cake **154** can be washed for as many times as required to develop a minimum concentration of metal catalyst in the washed cake **146** before discharging the washed cake **146** from the filter device.

A suitable pressure filter which can be adapted to the requirements of the instant invented process is a BHS-FEST^{™} rotary drum pressure filter, BHS-WERK, Sonthofen, D-8972, Sonthofen, West Germany, although other pressure filters which can accomplish the required operation can be used. Devices that can be used in the solid-liquid separation zone include **151,** are pressure belt filters, filter presses, centrifuges, pressure leaf filters, and cross-flow filters. The pressure filter can be operated at a temperature and pressure sufficient to obtain at least 80 wt% recovery of the metal catalyst from the solute of the mother liquor **147**. Preferably, the pressure filter can be operated at a temperature of 25°C to 160 °C, and a pressure of 1 × 10⁵ Pa (1 atmospheres) to 5 × 10⁶ Pa (50 atmospheres).

In the operation of the BHS-FEST^{™} filter, a rotary drum contains a series of filter cells located on the periphery of the rotating drum. As the drum rotates, the filter cells receive a super concentrated purge slurry **145** and a filter cake **154** builds to a requisite depth. The mother liquor **147** is produced by filtration of the super concentrated purge slurry **145**. Upon rotation of the drum, the filter cake **154** enters a washing zone **155** where a reservoir of wash feed **149** is built up over the filter cake **154** to a required depth: The applied pressure to the wash feed reservoir forces the water through the filter cake **154** to displace the solute (with dissolved metal catalyst) retained in the super concentrated purge slurry **145** to produce a washed cake **146**. Upon further rotation of the drum, the wash cycle can be repeated at least three more times if necessary in a counter current fashion, after which the system pressure is released with attendant temperature decrease to ambient conditions. Optionally, the washed cake **146** can be dewatered in a dewatering zone **157** with a vapor via conduit **152** to produce a dewatered cake **159** and a humid vapor **160**. The resultant dewatered cake **159** can then be discharged from the drum by any conventional means.

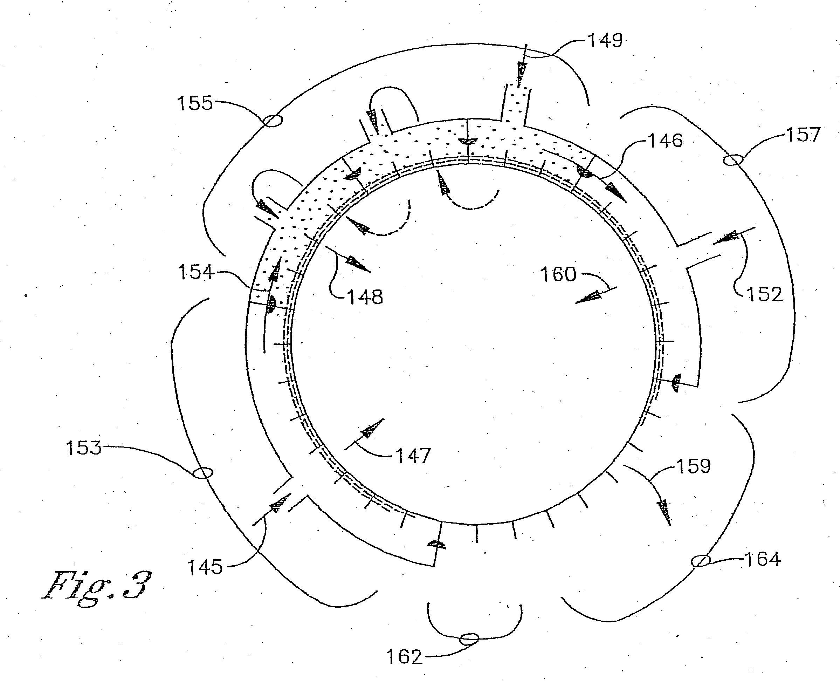

Figure 3 illustrates an embodiment of the invention where a rotary pressure drum filter is utilized as the process filtration device. In an embodiment of the invention, the rotary drum pressure filter comprises a filtration zone **153**, a wash zone **155**, optionally, a dewatering zone **157**, a discharge zone **164** and a cloth wash zone **162**. The cloth wash zone shown in Figure 3 is an embodiment of the invention where the rotary pressure drum filter comprises a cloth wash zone **162** where the filters are washed after discharge of the dewatered cake **159**.

The wash filtrate **148** is produced by displacement washing the filter cake with the wash feed **149**. The filter cake **154** within the solid-liquid separation zone **151** undergoes extraction of metal catalyst by introduction of the wash feed **149** to form the wash filtrate **148**. In an embodiment of the invention, at least 80 wt% of the metal catalyst is recovered in the wash filtrate **148** and the mother liquor **147**. In an embodiment of the invention, at least 90 wt% of the metal catalyst is recovered in the wash filtrate 148 and the mother liquor **147**. The wash feed **149** comprises water and optionally an additional oxidation solvent.

Perhaps most surprisingly by utilizing water as a wash feed **149** at temperatures in the range of 20°C to 70°C, preferably 30°C to 50°C, sufficient corrosion metal is retained in the dewatered cake **159** wherein the need for corrosion metal removal by other means is eliminated. The dewatered cake **159** which represents solids stripped of metal catalyst can be disposed from the system.

Step (d) comprises subjecting the mother liquor **147** to evaporation in an evaporator zone **210** to produce a solvent rich vapor stream **202** and wash filtrate residue **201**.

The evaporator zone **210** comprises at least one evaporator. In an embodiment of the invention, the evaporator is operated at atmospheric or slightly super atmospheric conditions, generally from about 1 × 10⁵ Pa (1 atmosphere) to about 1 × 10⁶ Pa (10 atmospheres). The solvent rich vapor **202** comprises a majority of the water and solvent, and the wash filtrate residue **201** comprises the remainder of the water and solvent not removed from the mother liquor **147** and the majority of the catalyst. The evaporation removes 90 wt% to 99 wt% of the solvent and water from the combined stream in conduit **147**, typically acetic acid and water, which are present in the wash filtrate **148** and the majority of the benzoic acid in the mother liquor **147**. "Majority" as used herein means greater than 50% by weight.

Step (e) comprises subjecting the solvent rich vapor stream **202** to conventional distillation in distillation zone **220** to form a benzoic acid rich stream **203** and a solvent rich stream **204**.

The separation zone **220** comprises at least one liquid-vapor separator. In an embodiment of the invention, the separator operates at atmospheric or slightly super atmospheric conditions, generally from 1 × 10⁵ Pa (1 atmosphere) to 1 × 10⁶ Pa (10 atmospheres). The liquid-vapor separator comprises at least one theoretical vapor-liquid equilibrium stage. Examples of liquid-vapor separators include, flash condensers and distillation columns.

In an embodiment of the invention, the benzoic rich acid stream **203** has greater than 5 wt% benzoic acid. In another embodiment of the invention, the benzoic acid rich stream **203** has greater than 15 wt% benzoic acid. In another embodiment of the invention, the benzoic acid rich stream **203** has greater than 30 wt% benzoic acid. In another embodiment of the invention, the benzoic acid rich stream **203** has greater than 50 wt% benzoic acid. In another embodiment of the invention, the benzoic acid rich stream **203** comprises from about 5 wt% to 75 wt% benzoic acid. In another embodiment of the invention, the benzoic acid rich stream **203** comprises from about 5 wt% to 50 wt% benzoic acid. In another embodiment of the invention, the benzoic acid rich stream **203** comprises from about 5 wt% to 35 wt% benzoic acid. In another embodiment of the invention, the benzoic acid rich stream **203** comprises from about 15 wt% to 30 wt% benzoic acid.

Step (f) comprises optionally recycling at least a portion of the solvent rich stream **204** back to an oxidation reactor in an aromatic oxidation process.

At least a portion of the solvent rich stream can be recycled back to an oxidation reactor in the oxidation process. "At least a portion" can mean at least 5 wt%, at least 15 wt%, at least 30 wt%, at least 50 wt%, at least 75 wt%, or all of the solvent rich stream is recycled **204** back to an oxidation reactor.

An example of an aromatic oxidation process is disclosed US. Patent Application 10/156,312.

Although the composition of the various streams in the process varies depending on the process conditions, a typical composition of the streams, using a computer simulated model(ASPEN version 12.1) of the process, are shown in Tables 1a and 1 b. In Tables 1a and 1b, the components are shown in the left hand column, and the amount of these components in each stream in Figure 1 are shown in the number column corresponding to the number of the stream in Figure 1.

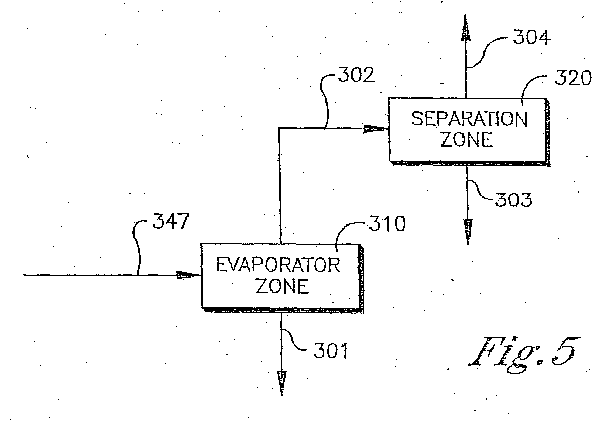

In another embodiment, a process is provided as shown in Figure 5, which is not according to the invention.

**Table 1A**

| ASPEN SIMULATION OF PROCESS | | | | | | | |
|---|---|---|---|---|---|---|---|
| | 101 MASS% | 104 MASS% | 105 MASS% | 144 MASS% | 145 MASS% | 146 MASS% | 147 MASS% |
| WATER | 7.7 | 8.9 | 4.3 | 6.0 | 2.7 | 20.0 | 3.5 |
| ACETIC ACID | 88.4 | 91.1 | 80.2 | 94.0 | 66.7 | 0.0 | 86.6 |
| TEREPHTHALIC ACID | 2.9 | 0.0 | 11.4 | 0.0 | 22.7 | 74.0 | 2.3 |
| OTHER ORGANICS | 0.6 | 0.1 | 2.0 | 0.0 | 4.0 | 5.9 | 3.1 |
| BENZOIC ACID | 0.3 | 0.0 | 1.1 | 0.0 | 2.1 | 0.0 | 2.8 |
| METALS AND BROMINE COMPLEX | 0.2 | 0.0 | 0.9 | 0.0 | 1.8 | 0.1 | 1.7 |
| | | | | | | | |
| TOTAL FLOW kg/hr | 10000 | 7500 | 2500 | 1241 | 1259 | 351 | 894 |
| TEMPERATURE C | 121.2 | 123.5 | 123.5 | 49.4 | 49.4 | 83.0 | 49.4 |

**Table 1B**

| | 148 MASS% | 149 MASS% | 201 MASS% | 202 MASS% | 203 MASS% | 204 MASS% |
|---|---|---|---|---|---|---|
| WATER | 89.8 | 100.0 | 0.0 | 3.7 | 1.2 | 3.9 |
| ACETIC ACID | 8.1 | 0.0 | 1.7 | 91.7 | 47.0 | 94.4 |
| TEREPHTHALIC ACID | 0.7 | 0.0 | 40.2 | 0.0 | 0.0 | 0.0 |
| OTHER ORGANICS | 0.3 | 0.0 | 27.1 | 1.6 | 20.3 | 0.5 |
| BENZOIC ACID | 0.3 | 0.0 | 0.7 | 2.9 | 31.5 | 1.2 |
| METALS AND BROMINE COMPLEX | 0.9 | 0.0 | 30.2 | 0.0 | 0.1 | 0.0 |
| | | | | | | |
| TOTAL FLOW kg/hr | 801 | 787 | 51 | 843 | 48 | 795 |
| TEMPERATURE C | 60.0 | 70.9 | 272.1 | 272.1 | 159.5 | 159.5 |

## Claims

1. A process to produce a benzoic acid rich stream, said process comprising:
(a) subjecting an oxidizer purge stream to evaporation in a main evaporator zone to produce a vapor stream and a super concentrated purge slurry;
(b) filtering said super concentrated purge slurry in a solid-liquid separation zone to form a filter cake and a mother liquor wherein said solid-liquid separation zone comprises at least one device selected from the group consisting of pressure belt filters, filter presses, centrifuges, pressure leaf filters, and cross-flow filters;
(c) washing said filter cake with a wash feed in said solid-liquid separation zone to form a washed cake and a wash filtrate;
(d) subjecting said mother liquor to evaporation in an evaporator zone to form a solvent rich vapor; and
(e) subjecting said solvent rich vapor to distillation in a separation zone to form a solvent rich stream and said benzoic acid rich stream.

2. The process according to claim 1 wherein 50 wt% to 80 wt% of said solvent and water is removed from said oxidizer purge stream in step (a).

3. The process according to claim 1 wherein 75 wt% to 99 wt% of said solvent and water is removed from said oxidizer purge stream in step (a).

4. The process according to claim 1 wherein 85 wt% to 99 wt% of said solvent and water is removed from said oxidizer purge stream in step (a).

5. The process according to claims 1, 2, 3, or 4 wherein said wash feed is added to said solid-liquid separation zone at a temperature range of 20°C to 100°C.

6. The process according to claim 5 wherein said wash feed is added to said solid-liquid separation zone at a temperature range of 30°C to 50°C.

7. The process according to claim 1 wherein said benzoic acid rich stream comprises benzoic acid in an amount greater than 30% by weight.

8. The process according to claim 1 wherein said main evaporator zone comprises at least one evaporator operated at a temperature of 20°C to 70°C.

9. The process according to claim 1 wherein said solid-liquid separation zone comprises at least one pressure filtration device.

10. The process according to claim 9 wherein said pressure filtration device operates at a temperature between 25°C to 160°C.

11. The process according to claim 9 wherein said pressure filtration device is operated at a pressure of 1x10⁵ Pa (1 atmosphere) to 5x10⁶ Pa (50 atmospheres).

12. The process according to claim 10 or 11 wherein said pressure filtration device comprises at least one filter cell and wherein at least one filter cell accumulates at least 0.64 cm (0.25 inch) in depth of said filter cake.

13. The process according to claim 10 or 11 wherein said pressure filtration device comprises at least one filter cell and wherein at least one filter cell accumulates at least 1.3 cm (0.5 inch) in depth of said filter cake.

14. The process according to claim 10 or 11 wherein said pressure filtration device comprises at least one filter cell and wherein at least one filter cell accumulates at least 3 cm (1 inch) in depth of said filter cake.

15. The process according to claim 10 or 11 wherein said wash feed forms a reservoir over said filter cake which is at least 0.64 cm (0.25 inch) in depth.

16. The process according to claim 10 or 11 wherein said pressure filtration device operates at a temperature between 25°C to 160°C

17. The process according to claim 16 wherein said pressure filtration device is operated at a pressure of 1x10⁵ Pa (1 atmosphere) to 5x10⁶ Pa (50 atmospheres).

18. The process according to claim 17 wherein said drying results in said dewatered cake having a moisture content from 10 wt% to 50 wt%.

19. The process according to claim 10 or 11 wherein said pressure filtration device is a rotary pressure drum filter.

20. The process according to claim 19 wherein said rotary pressure drum filter is operated at a pressure of 1x10⁵ Pa (1 atmosphere) to 5x10⁵ Pa (5 atmospheres).

21. The process according to claim 1 wherein said washing is counter current.

22. The process of claim 1, wherein step (a) comprises:
(a1) subjecting the oxidizer purge stream to evaporation in a first evaporator zone to produce a vapor stream and a concentrated purge slurry; and
(a2) subjecting said concentrated purge slurry to evaporation in a second evaporator zone to form a solvent rich stream and the super concentrated purge slurry.

23. The process according to claim 22 wherein 50 wt% to 80 wt% of said solvent and water is removed from said oxidizer purge stream in step (a).

24. The process according to claim 22 wherein 75 wt% to 99 wt% of said solvent and water is removed from said oxidizer purge stream in step (a) and step (b) combined.

25. The process according to claim 22 wherein 85 wt% to 99 wt% of said solvent and water is removed from said oxidizer purge stream in step (a) and step (b) combined.

26. The process according to claim 22 wherein 90 wt% to 99 wt% of said solvent and water is removed from said oxidizer purge stream in step (a) and step (b) combined.

27. The process according to claims 22, 23, 24, or 25 wherein said wash feed is added to said solid-liquid separation zone at a temperature range of 20°C to 100°C.

28. The process according to claim 22 wherein said wash feed is added to said solid-liquid separation zone at a temperature range of 30°C to 50°C.

29. The process according to claim 22 wherein said benzoic acid rich stream comprises benzoic acid in an amount greater than 30% by weight.

30. The process according to claim 22 wherein said second evaporator zone comprises an evaporator operated at a temperature of 20°C to 70°C.

31. The process according to claim 22 wherein said second evaporator zone comprises at least one evaporator that is operated at vacuum conditions.

32. The process according to claim 30 wherein said second evaporator zone comprises an evaporator that is operated at vacuum conditions.

33. The process according to claim 22 wherein said at least one device is a pressure filtration device and said pressure filtration device operates at a temperature between 25°C to 160°C.

34. The process according to claim 22 wherein said at least one device is a pressure filtration device and said pressure filtration device is operated at a pressure of 1x10⁵ Pa (1 atmosphere) to 5x10⁶ Pa (50 atmospheres).

35. The process according to claim 33 or 34 wherein said pressure filtration device comprises at least one filter cell and wherein at least one filter cell accumulates at least 0.64 cm (0.25 inch) in depth of said filter cake.

36. The process according to claim 33 or 34 wherein said pressure filtration device comprises at least one filter cell and wherein at least one filter cell accumulates at least 1.3 cm (0.5 inch) in depth of said filter cake.

37. The process according to claim 33 or 34 wherein said pressure filtration device comprises at least one filter cell and wherein at least one filter cell accumulates at least 3 cm (1 inch) in depth of said filter cake.

38. The process according to claim 33 or 34 wherein said wash feed forms a reservoir over said filter cake which is at least 0.64 cm (0.25 inch) in depth.

39. The process according to claim 33 or 34 wherein said pressure filtration device operates at a temperature between 25°C to 160°C.

40. The process according to claim 39 wherein said pressure filtration device is operated at a pressure of 1x10⁵ Pa (1 atmosphere) to 5x10⁶ Pa (50 atmospheres).

41. The process according to claim 40 wherein said dewatering results in said dewatered cake having a moisture content from 10 wt% to 50 wt%.

42. The process according to claim 33 or 34 wherein said pressure filtration device is a rotary pressure drum filter.

43. The process according to claim 42 wherein said rotary pressure drum filter is operated at a pressure of 1x10⁵ Pa (1 atmosphere) to 5x10⁵ Pa (5 atmospheres).

## Patentansprüche

1. Ein Verfahren zum Herstellen eines benzoesäurereichen Stroms, besagtes Verfahren umfassend:
(a) Unterziehen eines Oxidationsmittelspülstroms der Verdampfung in einer Hauptverdampferzone um einen Dampfstrom und einen hochkonzentrierten Spülschlamm zu bilden;
(b) Filtrieren des besagten hochkonzentrierten Spülschlamms in einer fest/flüssig-Abtrennzone, um einen Filterkuchen und eine Mutterlauge zu bilden, wobei besagte fest/flüssig-Abtrennzone mindestens eine Vorrichtung ausgewählt aus der Gruppe bestehend aus Druckbandfiltern, Filterpressen, Zentrifugen, Druck-Blatt-Filtern, und Cross-Flow-Filtern aufweist.
(c) Waschen des besagten Filterkuchens mit einer Waschbeschickung in besagter fest/flüssig-Abtrennzone um einen gewaschenen Kuchen und ein gewaschenes Filtrat zu bilden;
(d) Unterziehen der besagten Mutterlauge der Verdampfung in einer Verdampfungszone um einen lösungsmittelreichen Dampf zu bilden,
(e) Unterziehen des besagten lösungsmittelreichen Dampfes der Destillation in einer Abtrennzone um einen lösungsmittelreichen Strom und besagten benzoesäurereichen Strom zu bilden.

2. Das Verfahren gemäß Anspruch 1, wobei 50 Gew.-% bis 80 Gew.-% des besagten Lösungsmittels und Wassers von besagtem Oxidationsmittelspülstrom in Schritt (a) entfernt wird.

3. Das Verfahren gemäß Anspruch 1, wobei 75 Gew.-% bis 99 Gew.-% des besagten Lösungsmittels und Wassers von besagtem Oxidationsmittelspülstrom in Schritt (a) entfernt wird.

4. Das Verfahren gemäß Anspruch 1, wobei 85 Gew.-% bis 99 Gew.-% des besagten Lösungsmittels und Wassers von besagtem Oxidationsmittelspülstrom in Schritt (a) entfernt wird.

5. Das Verfahren gemäß Ansprüchen 1, 2, 3, oder 4, wobei besagte Waschbeschickung zu besagter fest/flüssig-Abtrennzone in einem Temperaturbereich von 20°C bis 100°C hinzugefügt wird.

6. Das Verfahren gemäß Anspruch 5, wobei besagte Waschbeschickung zu besagter fest/flüssig-Abtrennzone in einem Temperaturbereich von 30°C bis 50°C hinzugefügt wird.

7. Das Verfahren gemäß Anspruch 1, wobei besagter benzoesäurereicher Strom Benzoesäure in einer größeren Menge als 30 Gewichtsprozent aufweist.

8. Das Verfahren gemäß Anspruch 1, wobei besagte Hauptverdampferzone mindestens einen Verdampfer aufweist, der bei einer Temperatur von 20°C bis 70°C betrieben wird.

9. Das Verfahren gemäß Anspruch 1, wobei besagte fest/flüssig-Abtrennzone mindestens eine Druckfiltrationsvorrichtung aufweist.

10. Das Verfahren gemäß Anspruch 9, wobei besagte Druckfiltrationsvorrichtung bei einer Temperatur zwischen 25°C und 160°C arbeitet.

11. Das Verfahren gemäß Anspruch 9, wobei besagte Druckfiltrationsvorrichtung bei einem Druck von 1x10⁵ Pa (1 Atmosphäre) bis 5x10⁶ Pa (50 Atmosphären) betrieben wird.

12. Das Verfahren gemäß Anspruch 10 oder 11, wobei besagte Druckfiltrationsvorrichtung mindestens eine Filterzelle aufweist und wobei mindestens eine Filterzelle mindestens 0,64 cm (0,25 Zoll) in Tiefe des besagten Filterkuchens anhäuft.

13. Das Verfahren gemäß Anspruch 10 oder 11, wobei besagte Druckfiltrationsvorrichtung mindestens eine Filterzelle aufweist und wobei mindestens eine Filterzelle mindestens 1,3 cm (0,5 Zoll) in Tiefe des besagten Filterkuchens anhäuft.

14. Das Verfahren gemäß Anspruch 10 oder 11, wobei besagte Druckfiltrationsvorrichtung mindestens eine Filterzelle aufweist und wobei mindestens eine Filterzelle mindestens 3 cm (1 Zoll) in Tiefe des besagten Filterkuchens anhäuft.

15. Das Verfahren gemäß Anspruch 10 oder 11, wobei besagte Waschbeschickung ein Reservoir über besagtem Filterkuchen bildet, welches mindestens 0,64 cm (0,25 Zoll) tief ist.

16. Das Verfahren gemäß Anspruch 10 oder 11, wobei besagte Druckfiltrationsvorrichtung bei einer Temperatur zwischen 25°C bis 160°C arbeitet.

17. Das Verfahren gemäß Anspruch 16, wobei besagte Druckfiltrationsvorrichtung bei einem Druck von 1x10⁵ Pa (1 Atmosphäre) bis 5x10⁶ Pa (50 Atmosphären) betrieben wird.

18. Das Verfahren gemäß Anspruch 17, wobei das besagte Trocknen in besagtem entwässerten Kuchen, aufweisend einen Feuchtigkeitsgehalt von 10 Gew.-% bis 50 Gew.-%, resultiert.

19. Das Verfahren gemäß Anspruch 10 oder 11, wobei besagte Druckfiltrationsvorrichtung ein Rotationsdruck-Trommelfilter ist.

20. Das Verfahren gemäß Anspruch 19, wobei besagter Rotationsdruck-Trommelfilter bei einem Druck von 1x10⁵ Pa (1 Atmosphäre) bis 5x10⁵ Pa (5 Atmosphären) betrieben wird.

21. Das Verfahren gemäß Anspruch 1, wobei das besagte Waschen gegenstromartig stattfindet.

22. Das Verfahren gemäß Anspruch 1, wobei Schritt (a) aufweist:
(a1) Unterziehen des Oxidationsmittelspülstroms der Verdampfung in einer ersten Verdampferzone um einen Dampfstrom und einen konzentrierten Spülschlamm herzustellen; und
(a2) Unterziehen des besagten Spülschlamms der Verdampfung in einer zweiten Verdampferzone um einen lösungsmittelreichen Strom und den hochkonzentrierten Spülschlamm zu bilden.

23. Das Verfahren gemäß Anspruch 22, wobei 50 Gew.-% bis 80 Gew.-% des besagten Lösungsmittels und Wassers von besagtem Oxidationsmittelspülstrom in Schritt (a) entfernt wird.

24. Das Verfahren gemäß Anspruch 22, wobei 75 Gew.-% bis 99 Gew.-% des besagten Lösungsmittels und Wassers von besagtem Oxidationsmittelspülstrom in Schritt (a) und Schritt (b) zusammen entfernt wird.

25. Das Verfahren gemäß Anspruch 22, wobei 85 Gew.-% bis 99 Gew.-% des besagten Lösungsmittels und Wassers von besagtem Oxidationsmittelspülstrom in Schritt (a) und Schritt (b) zusammen entfernt wird.

26. Das Verfahren gemäß Anspruch 22, wobei 90 Gew.-% bis 99 Gew.-% des besagten Lösungsmittels und Wassers von besagtem Oxidationsmittelspülstrom in Schritt (a) und Schritt (b) zusammen entfernt wird.

27. Das Verfahren gemäß Ansprüchen 22, 23, 24, oder 25, wobei besagte Waschbeschickung zu besagter fest/flüssig-Abtrennzone in einem Temperaturbereich von 20°C bis 100°C hinzugefügt wird.

28. Das Verfahren gemäß Anspruch 22, wobei besagte Waschbeschickung zu besagter fest/flüssig-Abtrennzone in einem Temperaturbereich von 30°C bis 50°C hinzugefügt wird.

29. Das Verfahren gemäß Anspruch 22, wobei besagter benzoesäurereicher Strom Benzoesäure in einer größeren Menge als 30 Gewichtsprozent aufweist.

30. Das Verfahren gemäß Anspruch 22, wobei besagte zweite Verdampferzone einen Verdampfer aufweist, der bei einer Temperatur von 20°C bis 70°C betrieben wird.

31. Das Verfahren gemäß Anspruch 22, wobei besagte zweite Verdampferzone mindestens einen Verdampfer aufweist, der bei Vakuumbedingungen betrieben wird.

32. Das Verfahren gemäß Anspruch 30, wobei besagte zweite Verdampferzone einen Verdampfer aufweist, der bei Vakuumbedingungen betrieben wird.

33. Das Verfahren gemäß Anspruch 22, wobei besagte mindestens eine Vorrichtung eine Druckfiltrationsvorrichtung ist und besagte Druckfiltrationsvorrichtung bei einer Temperatur zwischen 25°C und 160°C arbeitet.

34. Das Verfahren gemäß Anspruch 22, wobei besagte mindestens eine Vorrichtung eine Druckfiltrationsvorrichtung ist und besagte Druckfiltrationsvorrichtung bei einem Druck von 1x10⁵ Pa (1 Atmosphäre) bis 5x10⁶ Pa (50 Atmosphären) betrieben wird.

35. Das Verfahren gemäß Anspruch 33 oder 34, wobei besagte Druckfiltrationsvorrichtung mindestens eine Filterzelle aufweist und wobei mindestens eine Filterzelle mindestens 0,64 cm (0,25 Zoll) in Tiefe des besagten Filterkuchens anhäuft.

36. Das Verfahren gemäß Anspruch 33 oder 34, wobei besagte Druckfiltrationsvorrichtung mindestens eine Filterzelle aufweist und wobei mindestens eine Filterzelle mindestens 1,3 cm (0,5 Zoll) in Tiefe des besagten Filterkuchens anhäuft.

37. Das Verfahren gemäß Anspruch 33 oder 34, wobei besagte Druckfiltrationsvorrichtung mindestens eine Filterzelle aufweist und wobei mindestens eine Filterzelle mindestens 3 cm (1 Zoll) in Tiefe des besagten Filterkuchens anhäuft.

38. Das Verfahren gemäß Anspruch 33 oder 34, wobei besagte Waschbeschickung ein Reservoir über besagtem Filterkuchen bildet, welches mindestens 0,64 cm (0,25 Zoll) tief ist.

39. Das Verfahren gemäß Anspruch 33 oder 34, wobei besagte Druckfiltrationsvorrichtung bei einer Temperatur zwischen 25°C bis 160°C arbeitet.

40. Das Verfahren gemäß Anspruch 39, wobei besagte Druckfiltrationsvorrichtung bei einem Druck von 1x10⁵ Pa (1 Atmosphäre) bis 5x10⁶ Pa (50 Atmosphären) betrieben wird.

41. Das Verfahren gemäß Anspruch 40, wobei besagtes Entwässern in besagtem entwässerten Kuchen, aufweisend einen Feuchtigkeitsgehalt von 10 Gew.-% bis 50 Gew.-%, resultiert.

42. Das Verfahren gemäß Anspruch 33 oder 34, wobei besagte Druckfiltrationsvorrichtung ein Rotationsdruck-Trommelfilter ist.

43. Das Verfahren gemäß Anspruch 42, wobei besagter Rotationsdruck-Trommelfilter bei einem Druck von 1x10⁵ Pa (1 Atmosphäre) bis 5x10⁵ Pa (5 Atmosphären) betrieben wird.

## Revendications

1. Procédé pour produire un courant riche en acide benzoïque, ledit procédé comprenant :
(a) l'exposition d'un courant de purge de réacteur d'oxydation à une évaporation dans une zone d'évaporateur principale pour produire un courant de vapeur et une suspension de purge superconcentrée ;
(b) la filtration de ladite suspension de purge superconcentrée dans une zone de séparation solide-liquide pour former un gâteau de filtration et une liqueur mère où ladite zone de séparation solide-liquide comprend au moins un dispositif choisi dans le groupe consistant en les filtres à bande sous pression, les filtres-presses, les centrifugeuses, les filtres à feuilles sous pression et les filtres à courant transversal ;
(c) le lavage dudit gâteau de filtration avec une charge de lavage dans ladite zone de séparation solide-liquide pour former un gâteau lavé et un filtrat de lavage ;
(d) l'exposition de ladite liqueur mère à une évaporation dans une zone d'évaporateur pour former une vapeur riche en solvant ; et
(e) l'exposition de ladite vapeur riche en solvant à une distillation dans une zone de séparation pour former un courant riche en solvant et ledit courant riche en acide benzoïque.

2. Procédé selon la revendication 1 où 50 % en poids à 80 % en poids dudit solvant et de l'eau sont retirés dudit courant de purge de réacteur d'oxydation dans l'étape (a).

3. Procédé selon la revendication 1 où 75 % en poids à 99 % en poids dudit solvant et de l'eau sont retirés dudit courant de purge de réacteur d'oxydation dans l'étape (a).

4. Procédé selon la revendication 1 où 85 % en poids à 99 % en poids dudit solvant et de l'eau sont retirés dudit courant de purge de réacteur d'oxydation dans l'étape (a).

5. Procédé selon la revendication 1, 2, 3 ou 4, où ladite charge de lavage est ajoutée à ladite zone de séparation solide-liquide dans une plage de température de 20°C à 100°C.

6. Procédé selon la revendication 5, où ladite charge de lavage est ajoutée à ladite zone de séparation solide-liquide dans une plage de température de 30°C à 50°C.

7. Procédé selon la revendication 1, où ledit courant riche en acide benzoïque comprend de l'acide benzoïque en une quantité supérieure à 30 % en poids.

8. Procédé selon la revendication 1, où ladite zone d'évaporateur principale comprend au moins un évaporateur fonctionnant à une température de 20°C à 70°C.

9. Procédé selon la revendication 1, où ladite zone de séparation solide-liquide comprend au moins un dispositif de filtration sous pression.

10. Procédé selon la revendication 9, où ledit dispositif de filtration sous pression fonctionne à une température entre 25°C et 160°C.

11. Procédé selon la revendication 9, où ledit dispositif de filtration sous pression fonctionne à une pression de 1 x 10⁵ Pa (1 atmosphère) à 5 x 10⁶ Pa (50 atmosphères).

12. Procédé selon la revendication 10 ou 11, où ledit dispositif de filtration sous pression comprend au moins une cellule de filtration et où au moins une cellule de filtration accumule au moins 0,64 cm (0,25 pouce) en profondeur dudit gâteau de filtration.

13. Procédé selon la revendication 10 ou 11, où ledit dispositif de filtration sous pression comprend au moins une cellule de filtration et où au moins une cellule de filtration accumule au moins 1,3 cm (0,5 pouce) en profondeur dudit gâteau de filtration.

14. Procédé selon la revendication 10 ou 11, où ledit dispositif de filtration sous pression comprend au moins une cellule de filtration et où au moins une cellule de filtration accumule au moins 3 cm (1 pouce) en profondeur dudit gâteau de filtration.

15. Procédé selon la revendication 10 ou 11, où ladite charge de lavage forme un réservoir au-dessus dudit gâteau de filtration qui est d'au moins 0,64 cm (0,25 pouce) en profondeur.

16. Procédé selon la revendication 10 ou 11, où ledit dispositif de filtration sous pression fonctionne à une température entre 25°C et 160°C.

17. Procédé selon la revendication 16, où ledit dispositif de filtration sous pression fonctionne à une pression de 1 x 10⁵ Pa (1 atmosphère) à 5 x 10⁶ Pa (50 atmosphères).

18. Procédé selon la revendication 17, où ledit séchage conduit à ce que ledit gâteau déshydraté a une teneur en humidité de 10 % en poids à 50 % en poids.

19. Procédé selon la revendication 10 ou 11, où ledit dispositif de filtration sous pression est un filtre à tambour rotatif sous pression.

20. Procédé selon la revendication 19, où ledit filtre à tambour rotatif sous pression fonctionne à une pression de 1 x 10⁵ Pa (1 atmosphère) à 5 x 10⁵ Pa (5 atmosphères).

21. Procédé selon la revendication 1, où ledit lavage est à contre-courant.

22. Procédé selon la revendication 1, où l'étape (a) comprend :
(a1) l'exposition du courant de purge de réacteur d'oxydation à une évaporation dans une première zone d'évaporateur pour produire un courant de vapeur et une suspension de purge concentrée ; et
(a2) l'exposition de ladite suspension de purge concentrée à une évaporation dans une seconde zone d'évaporateur pour former un courant riche en solvant et la suspension de purge superconcentrée.

23. Procédé selon la revendication 22, où 50 % en poids à 80 % en poids dudit solvant et de l'eau sont retirés dudit courant de purge de réacteur d'oxydation dans l'étape (a).

24. Procédé selon la revendication 22, où 75 % en poids à 99 % en poids dudit solvant et de l'eau sont retirés dudit courant de purge de réacteur d'oxydation dans l'étape (a) et l'étape (b) combinées.

25. Procédé selon la revendication 22, où 85 % en poids à 99 % en poids dudit solvant et de l'eau sont retirés dudit courant de purge de réacteur d'oxydation dans l'étape (a) et l'étape (b) combinées.

26. Procédé selon la revendication 22, où 90 % en poids à 99 % en poids dudit solvant et de l'eau sont retirés dudit courant de purge de réacteur d'oxydation dans l'étape (a) et l'étape (b) combinées.

27. Procédé selon la revendication 22, 23, 24 ou 25, où ladite charge de lavage est ajoutée à ladite zone de séparation solide-liquide dans une plage de température de 20°C à 100°C.

28. Procédé selon la revendication 22, où ladite charge de lavage est ajoutée à ladite zone de séparation solide-liquide dans une plage de température de 30°C à 50°C.

29. Procédé selon la revendication 22, où ledit courant riche en acide benzoïque comprend de l'acide benzoïque en une quantité supérieure à 30 % en poids.

30. Procédé selon la revendication 22, où ladite seconde zone d'évaporateur comprend un évaporateur fonctionnant à une température de 20°C à 70°C.

31. Procédé selon la revendication 22, où ladite seconde zone d'évaporateur comprend au moins un évaporateur qui fonctionne dans des conditions de vide.

32. Procédé selon la revendication 30, où ladite seconde zone d'évaporateur comprend un évaporateur qui fonctionne dans des conditions de vide.

33. Procédé selon la revendication 22, où ledit au moins un dispositif est un dispositif de filtration sous pression et ledit dispositif de filtration sous pression fonctionne à une température entre 25°C à 160°C.

34. Procédé selon la revendication 22, où ledit au moins un dispositif est un dispositif de filtration sous pression et ledit dispositif de filtration sous pression fonctionne à une pression de 1 x 10⁵ Pa (1 atmosphère) à 5 x 10⁶ Pa (50 atmosphères).

35. Procédé selon la revendication 33 ou 34, où ledit dispositif de filtration sous pression comprend au moins une cellule de filtration et où au moins une cellule de filtration accumule au moins 0,64 cm (0,25 pouce) en profondeur dudit gâteau de filtration.

36. Procédé selon la revendication 33 ou 34, où ledit dispositif de filtration sous pression comprend au moins une cellule de filtration et où au moins une cellule de filtration accumule au moins 1,3 cm (0,5 pouce) en profondeur dudit gâteau de filtration.

37. Procédé selon la revendication 33 ou 34, où ledit dispositif de filtration sous pression comprend au moins une cellule de filtration et où au moins une cellule de filtration accumule au moins 3 cm (1 pouce) en profondeur dudit gâteau de filtration.

38. Procédé selon la revendication 33 ou 34, où ladite charge de lavage forme un réservoir au-dessus dudit gâteau de filtration qui est d'au moins 0,64 cm (0,25 pouce) en profondeur.

39. Procédé selon la revendication 33 ou 34, où ledit dispositif de filtration sous pression fonctionne à une température entre 25°C et 160°C.

40. Procédé selon la revendication 39, où ledit dispositif de filtration sous pression fonctionne à une pression de 1 x 10⁵ Pa (1 atmosphère) à 5 x 10⁶ Pa (50 atmosphères).

41. Procédé selon la revendication 40, où ladite déshydratation conduit à ce que ledit gâteau déshydraté a une teneur en humidité de 10 % en poids à 50 % en poids.

42. Procédé selon la revendication 33 ou 34 où ledit dispositif de filtration sous pression est un filtre à tambour rotatif sous pression.

43. Procédé selon la revendication 42 où ledit filtre à tambour rotatif sous pression fonctionne à une pression de 1 x 10⁵ Pa (1 atmosphère) à 5 x 10⁵ Pa (5 atmosphères).
